# EUROPEAN PATENT APPLICATION

(11) **EP 1 889 542 A1**
(43) Date of publication of application: **20.02.2008**
(21) Application number: 06017023.0
(22) Date of filing: 16.08.2006
(51) Int. Cl.: A22C 5/00, G01N 33/12

(54) **Machine and method for the production of meat products**

(71) Applicant: CFS Slagelse A/S, 4200 Slagelse (DK)
(72) Inventor: Kofoed, Niels Peter, 4340 Tølløse (DK); Ditmarsen, Jan, 2600 Glostrup (DK)
(74) Representative: Wolff, Felix

(57) **Abstract**

The present invention relates to a food processing machine for the production of meat products and to a method for producing meat products.

## Description

The present invention relates to a food processing machine for the production of meat products and to a method for producing meat products.

Nowadays, the fat-content of meat products is usually indicated on the packages of the meat products. It is therefor necessary to determine the fat content. Exact fat analysis and setting of a certain fat-content are basic requirements for the production of meat on an industrial scale. Fat analysis is, for example, carried out by taking a sample from a mixer or from a conveyor and testing the sample in; a laboratory.

In WO 02/052257, a continuous fat analysis is proposed. The fat-content and the related mass flow of the meat which is transported on a conveyor belt is continuously determined, so that an overall fat content of a mixture can be calculated and controlled by feeding meat of higher or lower fat-content. One drawback of the described prior art is, that the average fat-content in a mixer does not assure that individual portions of meat comprise the same fat-content. Due to the inevitable variation of the fat content from portion to portion, the average fat content has to be kept well below the allowed or desired fat-content for the meat product. This means a considerable give-away of high-quality lean meat, which is economically unfavourable.

Accordingly, it is an object of the present invention to provide a food processing machine and method for the production of meat products, which avoids the disadvantages of the prior art.

The above objective is accomplished by a food processing machine for the production of meat products, comprising a pre-processing stage for providing portions of the meat product, an analysing means for determination of a fat-content of each portion and a post-processing stage, wherein the portions are processed, depending upon their individual fat-content.

It is an advantage of the food processing machine according to the invention, that the fat content of each individual portion of meat is determined. The post-processing of each individual portion depends upon its known fat-content. Even for unequal and alternating distribution of the fat-content over the multitude of portions, the production of meat products of undesirable high or low fat-content can be avoided.

The pre-processing stage in the sense of the present invention is any device or combination of devices for the preparation of meat products. Preferably, the pre-processing stage comprises a meat preparation station for providing a continuous meat product stream, which is, for example, an endless stream of minced meat or a bar for further processing in a slicer.

Furthermore preferable, the pre-processing stage comprises a separation station for separating portions from the product stream, which can be a cutter for separating portions from the endless stream of minced meat. Alternatively, the separation station can comprise a slicer for cutting slices off sausage bars or meat lumps, a certain number of slices forming a portion. It is an advantage of the present invention, that the fat-content of any portion of meat can be determined, regardless whether it comprises a certain amount of minced meat or, for example, an amount of sliced products, like sausage or bacon.

Preferably, the weight of the portions is known, for example the pre-processing stage comprises means for determining and/or calculating the weight of the portions. It is advantageous for the determination of the fat-content of the individual portion, if the weight of the portion is known.

The analysing means preferably comprises a contact-less fat analyser, based preferably on x-rays, infrared radiation or ultrasound. More preferable, the analysing means comprises a radiation source and a radiation detector, the portions being transportable through a clearance between the source and the detector, preferably on a transportation belt. Advantageously, the fat-analysis can be conducted on portions, which are transported on the conveyor belt. The portions can be packaged in a tray, for example, at the time of fat-analysis, or the portions can still be unpacked.

As fat analysis means, it is possible to use any fat analysis-means familiar to those skilled in the art. Preferably, however, the fat analysis means has a source of radiation, preferably having a plurality of energy steps, and a radiation detector. In a very particularly preferred embodiment, the source of radiation is an x-ray source and the radiation detector is an x-ray detector. Preference as source of radiation is likewise given to an infrared source and an ultrasound emitter. As radiation detector, to an infrared or ultrasound detector. The direction of transmission of radiation through the meat portion is preferably perpendicular to the transport direction, advantageously from above; In the case of fat analysis using an x-ray detector, an attenuation of the x-ray beam is measured, preferably in an energy range between 18 and 45 keV. Preferably, the distance between the x-ray source and the x-ray detector is 150 mm to 300 mm, particularly preferable 200 mm to 240 mm. The transmitted radiation region is preferably a truncated cone which advantageously has a taper angle of 10° to 40°, particularly advantageously 15° to 35°. Preferably, a microprocessor calculates the fat content and controls the x-ray source. The device is simple to manufacture and simple to integrate into a meat production process.

In a preferred embodiment of the inventive food processing machine, the post-processing stage comprises a sorting device for classifying the portions into predefined categories of fat-content. Categories of fat-content are, for example, customary fat-contents of meat products, given as a maximum fat-content of the portion. By sorting the portions after their individual and exactly determined fat-content, each final meat product is assigned to the appropriate category, which minimises the give-away of high-quality lean meat. The person skilled in the art understands that the average fat-content of the produced portions still depends upon the meat preparation in the pre-processing stage. The range of categories therefor can be rather narrow, for example with incremental steps of maximum fat-content between 2% and 5%. Advantageously the individual portions can be packaged after the sorting into categories, depending upon the category, or the portions in trays can be wrapped into an accordingly marked wrapping.

In a further preferred embodiment, the post-processing stage comprises a rejection device, by which portions of a fat-content, which is outside a predefined range, are rejected and preferably recycled back to the pre-processing stage. Advantageously, the production of meat products of objectionably high fat content, as well as of products with a uneconomically high give-away of lean meat is avoided by the present invention. The rejection device preferably comprises back-transportation-means, the rejection device directing portions, which do not meet the fat-content criteria, onto the back-transportation-means, which recycle the respective portions back to the pre-processing stage. A comparable back-transportation means is claimed in European Patent application no. 05 014 794. Advantageously, the time in which the separated meat portion is subjected to environmental conditions; e.g. elevated temperatures, is minimised.

There are a number of components known to the person skilled in the art in order to fulfil the function of directing a portion, which does not meet the fat-content criteria, onto the back-transportation-means. However, preferably, the rejection device comprises an endless belt, which can be flip-flopped between two different positions. One position is the normal production-position while the other position is the rejection-position.

Preferably, the sorting device as well comprises an endless belt which is movable into a number of different positions, the number of positions corresponding to the number of categories. More preferable, the sorting device and the rejection device are combined in only one device.

In a further preferred embodiment, the post-processing stage comprises a labelling means for providing a label to each portion, the label carrying information on the determined fat-content of the portion or information on a category of fat-content, to which the portion has been classified. Advantageously, the fat content on the label is the true fat-content or the true fat-content category of the individual portion of the meat product. Objections due to falsely labelled meat products are advantageously avoided.

Preferably the machine according to the present invention comprises packaging means for packaging the portions, for example into containers. The packaging means can be arranged upstream of the analysing means or upstream of the post-processing stage.

In a further aspect of the invention, it refers to a method for producing meat products, comprising the steps of:
- providing pre-processed portions of meat and
- determining a fat-content of each portion of meat,
wherein each portion of meat is further processed, depending upon its fat-content, in a post-processing stage.

Preferably, the portions are sorted into predefined categories of fat-content and/or rejected if the fat-content is outside a predefined range.

Furthermore preferred, the portions are labelled, the label carrying information on the determined fat-content of the portion or information on a category of fat-content, to which the portion has been classified.

These and other characteristics, features and advantages of the method and machine for the production of food products according to the present invention will become apparent from the following detailed description, taken in conjunction with the accompanying drawing, which illustrates, by way of example, the principles of the invention. The description is given for the sake of example only, without limiting the scope of the invention.

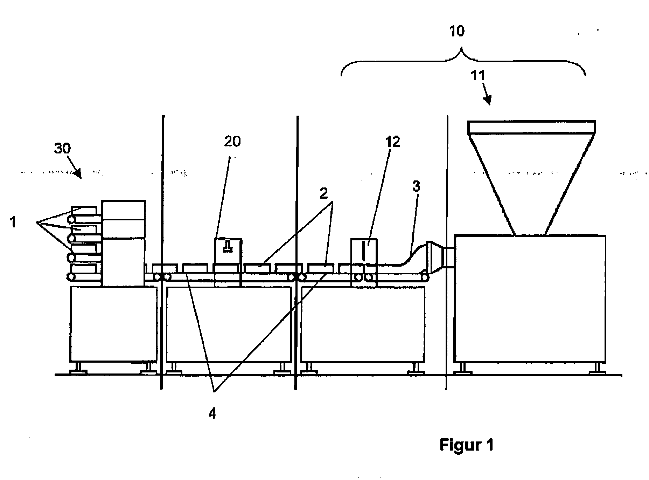

- **Figure 1**: shows a side elevation of a meat production machine according to the invention.

In **Figure 1** an exemplary embodiment of a machine for the production of meat products according to the present invention is depicted. A pre-processing stage 10 comprises a meat preparation station 11, which produces a product stream 3, which can be an endless stream of, for example, minced meat. The meat preparation station 11 is representative for any device or combination of devices which are used for the preparation of meat, such as grinding-, stuffing- or cooking devices. The pre-processing stage 10 further comprises a separation station 12, wherein the product stream 3 is separated into a multitude of individual portions 2. Transportation throughout the machine can be carried out by belt-conveyors 4. From the pre-processing stage 10, the portions 2 run through a analysing means 20 for the determination of the fat-content of each individual portion 2, and further to a post-processing stage 30. In the post-processing stage 30, the portions 2 are classified by a sorting device into categories of meat products 1, each category comprising a different maximum fat-content of the meat product 1. In the post-processing stage 30, the portions 2 are labelled according to their individual fat-content. Portions 2 with a fat content outside a predefined range are recycled back to the pre-processing stage 10.

## Claims

1. Food processing machine for the production of meat products (1), comprising a pre-processing stage (10) for providing portions (2) of the meat product, an analysing means (20) for determination of a fat-content of each portion (2) and a post-processing stage (30), wherein the portions (2) are processed, depending upon their individual fat-content.

2. Machine according to claim 1, **characterised in that** the pre-processing stage (10) comprises a meat preparation station (11) for providing a continuous meat product stream (3) and preferably a separation station (12) for separating portions (2) from the product stream (3).

3. Machine according to one of the preceding claims, **characterised in that** the analysing means (20) comprises a contact-less fat analyser, based preferably on x-rays, infrared radiation or ultrasound.

4. Machine according to one of the preceding claims, **characterised in that** the analysing means (20) comprises a radiation source and a radiation detector, the portions (2) being transportable through a clearance between the source and the detector, preferably on a transportation belt (4).

5. Machine according to one of the preceding claims, **characterised in that** the post-processing stage (30) comprises a sorting device for classifying the portions (2) into predefined categories of fat-content.

6. Machine according to one of the preceding claims, **characterised in that** the post-processing stage (30) comprises a rejection device, by which portions (2) of a fat-content, which is outside a predefined range, are rejected and preferably recycled back to the pre-processing stage (10).

7. Machine according to one of the preceding claims, **characterised in that** the post-processing stage (30) comprises a labelling means for providing a label to each portion, the label carrying information on the determined fat-content of the portion or information on a category of fat-content, to which the portion has been classified.

8. Method for producing meat products (1), comprising the steps of:
- providing pre-processed portions of meat (2) and
- determining a fat-content of each portion of meat,
wherein each portion of meat is further processed, depending upon its fat-content, in a post-processing stage (30).

9. Method according to claim 8, **characterised in that** the portions (2) are sorted into predefined categories of fat-content and/or rejected if the fat-content is outside a predefined range.

10. Method according to one of claims 8 or 9, **characterised in that** the portions (2) are labelled, the label carrying information on the determined fat-content of the portion or information on a category of fat-content, to which the portion has been classified.
